# EUROPÄISCHE PATENTANMELDUNG

(11) **EP 3 335 700 A1**
(43) Veröffentlichungstag der Anmeldung: **20.06.2018**
(21) Anmeldenummer: 16203722.0
(22) Anmeldetag: 13.12.2016
(51) Int. Cl.: A61K 9/20, A61K 31/00

(54) **FESTE PHARMAZEUTISCHE ORALE DARREICHUNGSFORM MIT VERLÄNGERTER WIRKSTOFFFREISETZUNG UMFASSEND MIRABEGRON**

(71) Anmelder: STADA ARZNEIMITTEL AG, 61118 Bad Vilbel (DE)
(72) Erfinder: SCHÖNBORN, Jessica, 61118 Bad Vilbel (DE)
(74) Vertreter: Kernebeck, Thomas

(57) **Zusammenfassung**

Die vorliegende Erfindung betrifft eine feste pharmazeutische orale Darreichungsform mit verlängerter Wirkstofffreisetzung, umfassend Mirabegron oder ein pharmazeutisch annehmbares Salz davon als Wirkstoff, wobei der Wirkstoff in einer verlängert freisetzenden Matrix verteilt enthalten ist. Um eine bioäquivalente Darreichungsform der gattungsgemäßen Art bereitzustellen, die verhältnismäßig einfach und damit kostengünstig hergestellt werden kann, wird vorgeschlagen, dass die Matrix einen Hydrogelbildner umfasst ausgewählt aus der Gruppe bestehend aus auf Poly(meth)acrylat-basierendem Polymer, Alginsäure, Alginat, Carrageenan und einer Mischung von zumindest zwei voneinander verschiedenen Hydroxypropylmethylcellulosen, wobei die Darreichungsform eine *in vitro*-Freisetzungsrate des Wirkstoffs, gemessen unter Anwendung der Drehkörbchen-Apparatur gemäß Eur. Ph. bei 100 U/min in 900 ml Phosphatpuffer mit einem pH-Wert von 6,8 bei einer Temperatur von 37 °C, von 20 % bis 40 % in 3 h, von 44 % bis 64 % in 5 h und von mehr als 80 % in 8,5 h aufweist.

## Beschreibung

Die vorliegende Erfindung betrifft eine feste pharmazeutische orale Darreichungsform mit verlängerter Wirkstofffreisetzung, umfassend Mirabegron oder ein pharmazeutisch annehmbares Salz davon als Wirkstoff, wobei der Wirkstoff in einer verlängert freisetzenden Matrix verteilt enthalten ist.

Mirabegron ist der internationale Freiname (INN (International Nonproprietary Name)) für 2-(2-Amino-1,3-thiazol-4-yl)-N-[4-(2-{[(2R)-2-hydroxy-2-phenylethyl]amino}ethyl)phenyl]acetamid und weist die folgende Strukturformel auf:

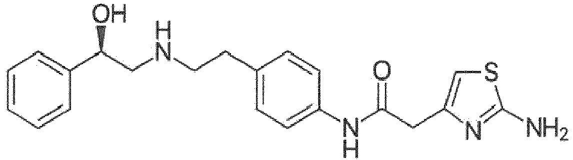

Mirabegron wird zur symptomatischen Therapie von imperativem Harndrang, erhöhter Miktionsfrequenz und/oder Dranginkontinenz eingesetzt, die bei Erwachsenen mit überaktiver Blase auftreten können.

Die Substanz Mirabegron wird original in der WO 99/20607 A1 offenbart, Verfahren zu deren Herstellung werden beispielsweise in der CN 103304511 A, der CN 103387500 A und der WO 2015/044965 A1 beschrieben.

Mirabegron ist ein starker Beta-3-Adrenozeptoragonist. In Modellversuchen zeigte sich, dass der Wirkstoff eine Entspannung der glatten Harnblasenmuskulatur bewirkt und so eine harnblasenentspannende Wirkung aufweist. Mirabegron erhöht das mittlere Entleerungsvolumen je Miktion und verringert die Häufigkeit von nicht zur Blasenentleerung führenden Blasenkontraktionen.

Patienten, die unter imperativem Harndrang leiden, wünschen eine nachhaltige Entlastung, d.h. der Harndrang soll im Verlauf einer kontinuierlichen natürlichen Harnblasenfüllung bis zur annäherend vollen Harnblase verringert werden. Diese Entlastung soll aber auch dauerhaft über ständig wiederkehrende Zyklen von Füllung und Entleerung gegeben sein. Dafür müssen Patienten über einen unbegrenzten Zeitraum dauerhaft entsprechende Medikamente einnehmen, wobei lang ausgedehnte Einnahmeintervalle im Hinblick auf die Patienten-Akzeptanz bevorzugt sind.

Mirabegron ist unter dem Handelsnamen Betmiga^{®} auf dem Markt. Dabei handelt es sich um Retardtabletten mit einer Dosierung von 25 mg bzw. 50 mg Mirabegron. Die retardierende Wirkung wird in besagten Tabletten im Wesentlichen mittels eines Gemisches von Polyethylenoxid und Hydroxypropylcellulose erreicht, zu dessen Stabilisierung Butylhydroxytoluol als Antioxidanz eingesetzt wird.

Aufgabe der vorliegenden Erfindung ist es, eine feste pharmazeutische orale Darreichungsform mit verlängerter Wirkstofffreisetzung, umfassend Mirabegron oder ein pharmazeutisch annehmbares Salz davon als Wirkstoff, wobei der Wirkstoff in einer verlängert freisetzenden Matrix verteilt enthalten ist, bereitzustellen, die bioäquivalent zu Betmiga^{®} ist und verhältnismäßig einfach und damit kostengünstig hergestellt werden kann.

Ausgehend von einer Darreichungsform der gattungsgemäßen Art wird diese Aufgabe dadurch gelöst, dass die Matrix einen Hydrogelbildner umfasst ausgewählt aus der Gruppe bestehend aus auf Poly(meth)acrylat-basierendem Polymer, Alginsäure, Alginat, Carrageenan und einer Mischung von zumindest zwei voneinander verschiedenen Hydroxypropylmethylcellulosen, wobei die Darreichungsform eine in *vitro*-Freisetzungsrate des Wirkstoffs, gemessen unter Anwendung der Drehkörbchen-Apparatur gemäß Eur. Ph. bei 100 U/min in 900 ml Phosphatpuffer mit einem pH-Wert von 6,8 bei einer Temperatur von 37 °C, von 20 % bis 40 % in 3 h, von 44 % bis 64 % in 5 h und von mehr als 80 % in 8,5 h aufweist.

Überraschenderweise wurde festgestellt, dass bei der erfindungsgemäßen Darreichungsform in der Matrix beispielsweise auf den Zusatz von Antioxidanzien zur selben verzichtet werden kann, so dass die erfindungsgemäße Darreichungsform verfahrenstechnisch verhältnismäßig einfach und damit kostengünstig hergestellt werden kann.

Ferner, da bei der erfindungsgemäßen Darreichungsform auf den Einsatz von Antioxidantien verzichtet werden kann, verfügt diese vorteilhafterweise auch über eine erhöhte Patientenakzeptanz.

Darüber hinaus konnte festgestellt werden, dass die erfindungsgemäße Darreichungform unter Verwendung der spezifizierten Hydrogelbildner in der Matrix mittels trockener Verfahren - das heißt ohne den Einsatz von Lösungsmitteln - hergestellt werden kann, was abermals die Kosten für die Herstellung vermindert.

Feste pharmazeutische orale Darreichungsformen mit verlängerter Wirkstofffreisetzung sind im Stand der Technik bekannt, so zum

Beispiel im Europäisches Arzneibuch, 8. Ausgabe, Grundwerk 2014, 8.0/0478 "Tabletten", S. 1203-1207, wo unter dem Stichwort "Tabletten mit veränderter Wirkstofffreisetzung" unter anderem von Tabletten mit verlängerter Wirkstofffreisetzung (Retard-Tabletten) die Rede ist.

Hydrogele, Hydrogelbildner und entsprechende Matrixformulierungen sind ebenfalls im Stand der Technik bekannt (siehe "Die Tablette", Handbuch der Entwicklung, Herstellung und Qualitätssicherung, A. Bauer-Brandl et al., Editio Cantor Verlag, 3. Auflage, 2012 (ISBN 978-3-87193-407-0)).

Die *in vitro*-Freisetzungsrate der erfindungsgemäßen Darreichungsform wird unter Anwendung der Drehkörbchen-Apparatur (Apparatur 1) und der entsprechenden Ausführungsvorschrift gemäß Eur.Ph. (Europäisches Arzneibuch, 8. Ausgabe, 7. Nachtrag, 2.9.3 "Wirkstofffreisetzung aus festen Arzneiformen", S. 7367-7375) bei 100 U/min in 900 ml Phosphatpuffer (34, 03 g Kaliumdihydrogenphosphat mit 112 ml 1,0 M NaOH mischen und mit demineralisiertem Wasser auffüllen auf 5000 ml) mit einem pH-Wert von 6,8 bei 37 °C bestimmt. Die Menge an freigesetztem Wirkstoff wird vorzugsweise mittels UV-Detektion bei einer Wellenlänge von 250 nm bestimmt.

Im Rahmen der vorliegenden Erfindung kann die erfindungsgemäße Darreichungsform Mirabegron nicht nur in Form der freien Base, sondern auch in Form eines pharmazeutisch annehmbaren Salzes davon enthalten. Pharmazeutisch annehmbare Salze von Mirabegron sind beispielsweise Säure-Additionssalze von Mirabegron, die durch Umsetzen von Mirabegron mit anorganischen oder organischen Säuren hergestellt werden können, wie etwa Salzsäure, Bromwasserstoffsäure, Schwefelsäure, Phosphorsäure, Milchsäure, Zitronensäure, Weinsäure, Bernsteinsäure, Maleinsäure oder Fumarsäure.

Gemäß einer bevorzugten Ausführungsform der erfindungsgemäßen Darreichungsform ist es vorgesehen, dass sich die voneinander verschiedenen Hydroxypropylmethylcellulosen hinsichtlich ihrer Viskosität voneinander unterscheiden. Erfindungsgemäß bevorzugt weisen die HPMC-Qualitäten eine (dynamische) Viskosität in einem Bereich von 6 bis 100000 mPa·s auf, weiter bevorzugt eine Viskosität in einem Bereich von 100 bis 100000 mPa·s, mehr bevorzugt eine Viskosität in einem Bereich von 100 bis 10000 mPa·s und noch mehr bevorzugt eine Viskosität in einem Bereich von 100 bis 4000 mPa·s. Die Viskosität wird erfindungsgemäß bevorzugt gemäß Eur.Ph. (Europäisches Arzneibuch, 8. Ausgabe, 6. Nachtrag, 8.6/0348 "Hypromellosen", S. 7595-7597) bestimmt.

Nach einer weiter bevorzugten Ausführungsform der erfindungsgemäßen Darreichungsform ist es vorgesehen, dass das Alginat ein Natriumalginat oder ein Calciumalginat ist.

Alginsäure (auch Algin) wird von Braunalgen und von einigen Bakterien (z. B. Azotobacter) gebildet. In den Algen stellt sie das strukturgebende Element der Zellwände dar. Sie eignet sich sowohl in behandelter als auch in unbehandelter Form als Hydrogelbildner.

Bei Carrageenan handelt es sich um langkettige Kohlenhydrate, die in Rotalgenzellen vorkommen. Dabei sind die Kohlenhydrate lineare anionische Hydrokolloide, die sich nach ihrer chemischen Struktur unterscheiden lassen.

Entsprechend einer weiter bevorzugten Ausführungsform der erfindungsgemäßen Darreichungsform ist es vorgesehen, dass das auf Poly(meth)acrylat-basierende Polymer ein Copolymer von Ethylacrylat und Methylmethacrylat ist oder ein Terpolymer von Ethylacrylat, Methylmethacrylat und einem Methacrylsäureester, insbesondere das Copolymer Polyethylacrylat-methylmethacrylat mit einem Ethylacrylat/Methylmethacrylat-Verhältnis von 2:1, beispielsweise Eudragit^{®} NE 30 D.

Gemäß einer weiter bevorzugten Ausführungsform der erfindungsgemäßen Darreichungsform ist es vorgesehen, dass die Matrix frei ist von Polyethylenoxid, wobei es noch mehr bevorzugt ist, wenn die gesamte Darreichungsform frei von Polyethylenoxid ist. In Polyethylenoxid - gleichbedeutend mit Polyethylenglykol - können Rückstände enthalten sein, die im Verdacht stehen gesundheitsgefährdend zu sein. Darüber hinaus ist Polyethylenoxid oxidationsempfindlich, was zu einer nachteiligen Veränderung des Freisetzungsprofils der Darreichungsform im Laufe der Zeit führen kann.

Entsprechend einer weiter bevorzugten Ausführungsform der erfindungsgemäßen Darreichungsform ist es vorgesehen, dass die Matrix frei von Antioxidantien ist, vorzugsweise die gesamte Darreichungsform. Unter Antioxidantien sollen chemische Verbindungen verstanden werden, die die Oxidation von chemischen Substanzen - beispielsweise Polyethylenoxid - verlangsamen oder verhindern. Der Verzicht von Antioxidantien erhöht die Medikamentenverträglichkeit und damit die Patientenakzeptanz. Beispiele für Antioxidantien, die ausgeschlossen sein sollen sind insbesondere Butylhydroxytoluol und Tocopherole.

Entsprechend einer weiter bevorzugten Ausführungsform der erfindungsgemäßen Darreichungsform ist es vorgesehen, dass das Gewichtsverhältnis von Wirkstoff zu dem Hydrogelbildner in einem Bereich von 50:1 bis 1:50 liegt, weiter bevorzugt in einem Bereich von 20:1 bis 1:20 und besonders bevorzugt in einem Bereich von 10:1 bis 1:10. Dadurch wird eine ausreichend verzögerte Freisetzung des Wirkstoffes sichergestellt.

Nach einer weiter bevorzugten Ausführungsform der erfindungsgemäßen Darreichungsform ist es vorgesehen, dass das Gewichtsverhältnis von Wirkstoff zu dem Hydrogelbildner in einem Bereich von 1:1 bis 1:50 liegt, weiter bevorzugt in einem Bereich von 1:2 bis 1:20 und besonders bevorzugt in einem Bereich von 1:3 bis 1:10. Dadurch wird ebenfalls eine ausreichend verzögerte Freisetzung des wirkstoffes sichergestellt.

In einer weiter bevorzugten Ausführungsform der erfindungsgemäßen Darreichungsform ist es vorgesehen, dass der Gehalt der Darreichungsform an Mirabegron bezogen auf die freie Base 1 bis 100 mg beträgt, mehr bevorzugt 25 mg bis 50 mg und noch mehr bevorzugt 25 mg oder 50 mg.

Entsprechend einer weiter bevorzugten Ausführungsform der erfindungsgemäßen Darreichungsform ist es vorgesehen, dass die verlängert freisetzende Matrix zumindest zu 10 Gew.-% aus dem Hydrogelbildner besteht, mehr bevorzugt zumindest zu 20 Gew.-%, weiter bevorzugt zumindest zu 30 Gew.-%, noch mehr bevorzugt zumindest zu 50 Gew.-% und noch mehr bevorzugt zumindest zu 70 Gew.-%.

Gemäß einer weiter bevorzugten Ausführungsform der erfindungsgemäßen Darreichungsform ist es vorgesehen, dass die Darreichungsform frei von pharmazeutischen Hilfsstoffen ist, die in Wasser basisch reagieren. Basisch reagierende Hilfsstoffe sind Hilfsstoffe, die bei Zusammenführung mit demineralisiertem Wasser bei 20 °C basisch reagieren, d.h., dass das Wasser nach der Zusammenführung einen pH-Wert von größer als 7 aufweist. Durch diese Maßnahme wird eine verhältnismäßig gute Löslichkeit des Wirkstoffes gewährleistet, insbesondere wenn Mirabegron in Form der freien Base eingesetzt wird.

In der erfindungsgemäßen Darreichungsform können pharmazeutisch annehmbare Hilfsstoffe erforderlich sein. So sind beispielsweise Verarbeitungshilfsmittel oder Bindemittel vorteilhaft, um Eigenschaften wie die Verarbeitbarkeit oder die mechanische Stabilität zu verbessern. In einer besonders bevorzugten Ausführungsform der vorliegenden Erfindung umfasst die Darreichungsform ein Füllmittel, ein Bindemittel, ein Sprengmittel, ein Schmiermittel und/oder ein Fließregulierungsmittel als pharmazeutisch annehmbaren Hilfsstoff.

Entsprechend einer weiter bevorzugten Ausführungsform der erfindungsgemäßen Darreichungsform ist es vorgesehen, dass die Darreichungsform eine Tablette ist, die optional mit einem Filmüberzug versehen ist. Tabletten besitzen im Gegensatz zu anderen Darreichungsformen eine verhältnismäßig hohe Patientenakzeptanz. Tabletten sind einzeldosierte feste Darreichungsformen, die unter Anwendung eines Pressdrucks aus Pulvern, Granulaten, oder Pulver/Granulat-Mischungen auf Tablettenpressen hergestellt werden können. Eine Tablette stellt in der Regel die Dosiseinheit dar, die für Patienten am leichtesten zu handhaben und darüber hinaus verhältnismäßig kostengünstig ist.

Nach einer weiter bevorzugten Ausführungsform der erfindungsgemäßen Darreichungsform ist es vorgesehen, dass die Tablette direktkomprimiert ist. Dabei wird das die Tablettenbestandteile enthaltende Pulver ohne weitere Vorbehandlung zu einer Tablette verpresst. Der Vorteil einer direktkomprimierten Tablette liegt darin, dass sie verhältnismäßig einfach herstellbar und damit kostengünstig ist.

Gemäß einer weiter bevorzugten Ausführungsform der erfindungsgemäßen Darreichungsform ist es vorgesehen, dass die

Tablette mittels eines den wirkstoff enthaltenden Granulats hergestellt ist. Durch diese Maßnahme kann eine mechanisch verhältnismäßig stabile Tablette erhalten werden. Hierbei ist es insbesondere bevorzugt, wenn der Wirkstoff zusammen mit dem Hydrogelbildner im Granulat vorliegt.

Die vorliegende Erfindung betrifft ferner ein Verfahren zur Herstellung der erfindungsgemäßen Darreichungsform, umfassend die Schritte des
(a) Vermischens des Wirkstoffes mit dem Hydrogelbildner unter Erhalt eines Gemisches;
(b) Verarbeiten des Gemisches aus Schritt (a) zu der Darreichungsform,
wobei das Verfahren unter Ausschluss des Einsatzes eines Lösungsmittels durchgeführt wird.

Das Merkmal, demgemäß das erfindungsgemäße Verfahren zur Herstellung der erfindungsgemäßen Darreichungsform ohne den Einsatz von Lösungsmitteln durchgeführt wird, ist so zu verstehen, dass der bereitgestellte Wirkstoff und der bereitgestellte Hydrogelbildner zu keinem Zeitpunkt der Fertigung der Darreichungsform mit einer Flüssigkeit in Kontakt gebracht werden. Dies bezieht sich jedoch nur auf den absichtlichen gezielten Einsatz von Flüssigkeiten/Lösungsmitteln und nicht etwa auf eine etwaige Restfeuchte, die dem Wirkstoff, dem Hydrogelbildner oder etwaigen pharmazeutischen Hilfsstoffen anhaftet. Derartige Verfahren werden im Stand der Technik auch als trockene Verfahren bezeichnet.

Das erfindungsgemäße Verfahren hat den Vorteil, dass die resultierende Darreichungsform sowohl durch eine verhältnismäßig hohe morphologische Stabilität des darin enthaltenen Wirkstoffs als auch durch eine verhältnismäßig hohe chemische Stabilität desselben gekennzeichnet ist.

Gemäß einer bevorzugten Ausführungsform des erfindungsgemäßen Verfahrens umfasst das Verfahren die Schritte
(a) Bereitstellen des Wirkstoffes und des Hydrogelbildners;
(b) Vermischen des Wirkstoffes und des Hydrogelbildners; und
(c) Direktverpressen des Gemisches aus Schritt (b) unter Erhalt eines Tablettenkerns.

Diese bevorzugte Ausführungsform des erfindungsgemäßen Verfahrens führt zu einer erfindungsgemäßen Darreichungsform, die besonders kostengünstig ist.

Entsprechend einer anderen bevorzugten Ausführungsform des erfindungsgemäßen Verfahrens umfasst das Verfahren die Schritte
(a) Bereitstellen des Wirkstoffes und des Hydrogelbildners;
(b) Vermischen des Wirkstoffes und des Hydrogelbildners unter Erhalt eines Gemisches;
(c) Trockengranulieren des Gemisches aus Schritt (b) unter Erhalt eines Trockengranulats; und
(d) Verpressen des Trockengranulats aus Schritt (c) unter Erhalt eines Tablettenkerns.

Diese bevorzugte Ausführungsform des erfindungsgemäßen Verfahrens führt zu erfindungsgemäßen Darreichungsformen, die eine verhältnismäßig hohe mechanische Festigkeit aufweisen. Gegebenenfalls werden die erhaltenen Tablettenkerne in üblicher Weise mit einem Filmüberzug versehen.

Die nachstehende Beschreibung bevorzugter Ausführungsbeispiele der erfindungsgemäßen Darreichungsform und des erfindungsgemäßen Verfahrens dienen der Erläuterung der Erfindung:

### Ausführungsbeispiel 1

Es wurden Tabletten mit der in Tabelle 1 angegebenen Zusammensetzung hergestellt:

**Tabelle 1:**

| Bestandteil | Menge pro Tablette | Funktion des Bestandteiles |
|---|---|---|
| Phase I | | |
| Alginsäure | 100 mg | Hydrogelbildner |
| Mirabegron | 25 mg | Wirkstoff |
| | | |
| Phase II | | |
| Siliciumdioxid, hochdisperses Aerosil^{®} 200 | 2 mg | Fließregulierungsmittel |
| Stearinsäure | 1 mg | Schmiermittel |
| | | |
| Endgewicht Tablettenkern | 128 mg | |
| Filmüberzug (Hypromellosen, TiO₂) | 3 mg | |
| | | |
| Endgewicht Tablette | 131 mg | |

Die Herstellung der Tabletten erfolgte, indem zunächst die Alginsäure als Hydrogelbildner und Mirabegron als Wirkstoff miteinander vermischt wurden unter Erhalt eines Gemisches.

Dieses Gemisch wurde mittels eines Walzenkompaktators komprimiert und anschließend gemahlen und gesiebt. Das so erhaltene Granulat wurde mit Siliciumdioxid als Fließregulierungsmittel und Stearinsäure als Schmiermittel vermischt und das so erhaltene Gemisch zu Tablettenkernen verpresst. Die so erhaltenen Tablettenkerne wurden mit einem handelsüblichen Filmüberzug auf Wasserbasis enthaltend Hypromellose und TiO₂ befilmt.

Die Filmtabletten weisen eine *in vitro*-Freisetzungsrate des Wirkstoffs von 20 % bis 40 % in 3 h, von 44 % bis 64 % in 5 h und von mehr als 80 % in 8,5 h auf.

### Ausführungsbeispiel 2

Es wurden Tabletten mit der in Tabelle 2 angegebenen Zusammensetzung hergestellt:

**Tabelle 2:**

| Bestandteil | Menge pro Tablette | Funktion des Bestandteiles |
|---|---|---|
| Phase I | | |
| Carrageenan | 100 mg | Hydrogelbildner |
| Mirabegron | 25 mg | Wirkstoff |
| | | |
| Phase II | | |
| Siliciumdioxid, hochdisperses Aerosil^{®} 200 | 2 mg | Fließregulierungsmittel |
| Stearinsäure | 1 mg | Schmiermittel |
| | | |
| Endgewicht Tablettenkern | 128 mg | |
| Filmüberzug (Hypromellose, TiO₂) | 3 mg | |
| Endgewicht Tablette | 131 mg | |

Die Herstellung der Tabletten erfolgte analog Ausführungsbeispiel 1.

Die Filmtabletten weisen eine in *vitro*-Freisetzungsrate des wirkstoffs von 20 % bis 40 % in 3 h, von 44 % bis 64 % in 5 h und von mehr als 80 % in 8,5 h auf.

### Ausführungsbeispiel 3

Es wurden Tabletten mit der in Tabelle 3 angegebenen Zusammensetzung hergestellt:

**Tabelle 3:**

| Bestandteil | Menge pro Tablette | Funktion des Bestandteiles |
|---|---|---|
| Phase I | | |
| Ethylacrylatmethylmethacrylat-Copolymer Eudragit^{®} NE 30 D | 100 mg | Hydrogelbildner |
| Mirabegron | 25 mg | Wirkstoff |
| | | |
| Phase II | | |
| Siliciumdioxid, hochdisperses Aerosil^{®} 200 | 2 mg | Fließregulierungsmittel |
| Stearinsäure | 1 mg | Schmiermittel |
| | | |
| Endgewicht Tablettenkern | 128 mg | |
| Filmüberzug (Hypromellose, TiO₂) | 3 mg | |
| Endgewicht Tablette | 131 mg | |

Die Herstellung der Tabletten erfolgte analog Ausführungsbeispiel 1.

Die Filmtabletten weisen eine in *vitro*-Freisetzungsrate des Wirkstoffs von 20 % bis 40 % in 3 h, von 44 % bis 64 % in 5 h und von mehr als 80 % in 8,5 h auf.

### Ausführungsbeispiel 4

Es wurden Tabletten mit der in Tabelle 4 angegebenen Zusammensetzung hergestellt:

**Tabelle 4:**

| Bestandteil | Menge pro Tablette | Funktion des Bestandteiles |
|---|---|---|
| Alginsäure | 100 mg | Hydrogelbildner |
| Mirabegron | 25 mg | Wirkstoff |
| Siliciumdioxid, hochdisperses Aerosil^{®} 200 | 2 mg | Fließregulierungsmittel |
| Stearinsäure | 1 mg | Schmiermittel |
| | | |
| Endgewicht Tablettenkern | 128 mg | |
| Filmüberzug (Hypromellose, TiO₂) | 3 mg | |
| | | |
| Endgewicht Tablette | 131 mg | |

Die Herstellung der Tabletten erfolgte, indem zunächst die Alginsäure als Hydrogelbildner, Mirabegron als Wirkstoff, Siliciumdioxid als Fließregulierungsmittel und Stearinsäure als Schmiermittel miteinander vermischt wurden unter Erhalt eines Gemisches. Dieses Gemisch wurde mittels einer herkömmlichen Tablettenpresse zu Tablettenkernen verpresst. Die so erhaltenen Tablettenkerne wurden mit einem handelsüblichen Filmüberzug auf Wasserbasis enthaltend Hypromellose und TiO₂ befilmt.

Die Filmtabletten weisen eine *in vitro*-Freisetzungsrate des Wirkstoffs von 20 % bis 40 % in 3 h, von 44 % bis 64 % in 5 h und von mehr als 80 % in 8,5 h auf.

### Ausführungsbeispiel 5

Es wurden Tabletten mit der in Tabelle 5 angegebenen Zusammensetzung hergestellt:

**Tabelle 5:**

| Bestandteil | Menge pro Tablette | Funktion des Bestandteiles |
|---|---|---|
| Carrageenan | 100 mg | Hydrogelbildner |
| Mirabegron | 25 mg | Wirkstoff |
| Siliciumdioxid, hochdisperses Aerosil^{®} 200 | 2 mg | Fließregulierungsmittel |
| Stearinsäure | 1 mg | Schmiermittel |
| | | |
| Endgewicht Tablettenkern | 128 mg | |
| Filmüberzug (Hypromellose, TiO₂) | 3 mg | |
| | | |
| Endgewicht Tablette | 131 mg | |

Die Tabletten wurden analog Ausführungsbeispiel 4 hergestellt.

Die Filmtabletten weisen eine *in vitro*-Freisetzungsrate des Wirkstoffs von 20 % bis 40 % in 3 h, von 44 % bis 64 % in 5 h und von mehr als 80 % in 8,5 h auf.

### Ausführungsbeispiel 6

Es wurden Tabletten mit der in Tabelle 6 angegebenen Zusammensetzung hergestellt:

**Tabelle 6:**

| Bestandteil | Menge pro Tablette | Funktion des Bestandteiles |
|---|---|---|
| Ethylacrylatmethylmethacrylat-Copolymer Eudragit^{®} NE 30 D | 100 mg | Hydrogelbildner |
| Mirabegron | 25 mg | Wirkstoff |
| Siliciumdioxid, hochdisperses Aerosil^{®} 200 | 2 mg | Fließregulierungsmittel |
| Stearinsäure | 1 mg | Schmiermittel |
| | | |
| Endgewicht Tablettenkern | 128 mg | |
| Filmüberzug (Hypromellose, TiO₂) | 3 mg | |
| | | |
| Endgewicht Tablette | 131 mg | |

Die Tabletten wurden analog Ausführungsbeispiel 4 hergestellt.

Die Filmtabletten weisen eine in *vitro*-Freisetzungsrate des Wirkstoffs von 20 % bis 40 % in 3 h, von 44 % bis 64 % in 5 h und von mehr als 80 % in 8,5 h auf.

### Ausführungsbeispiel 7

Es wurden Tabletten mit der in Tabelle 7 angegebenen Zusammensetzung hergestellt:

**Tabelle 7:**

| Bestandteil | Menge pro Tablette | Funktion des Bestandteiles |
|---|---|---|
| Hypromellose (Viskosität 100 mPa·s) | 17.5 mg | Hydrogelbildner |
| Hypromellose (Viskosität 4000 mPa·s) | 25 mg | Hydrogelbildner |
| Mirabegron | 25 mg | Wirkstoff |
| Mikrokristalline Cellulose | 57.5 mg | Füllmittel |
| Siliciumdioxid, hochdisperses Aerosil^{®} 200 | 2 mg | Fließregulierungsmittel |
| Stearinsäure | 1 mg | Schmiermittel |
| | | |
| Endgewicht Tablettenkern | 128 mg | |
| Filmüberzug (Hypromellose, TiO₂) | 3 mg | |
| | | |
| Endgewicht Tablette | 131 mg | |

Die Herstellung der Tabletten erfolgte, indem zunächst die Hypromellosen als Hydrogelbildner, Mirabegron als Wirkstoff, mikrokristalline Cellulose als Füllmittel, Siliciumdioxid als Fließregulierungsmittel und Stearinsäure als Schmiermittel miteinander vermischt wurden unter Erhalt eines Gemisches. Dieses Gemisch wurde mittels einer herkömmlichen Tablettenpresse zu Tablettenkernen verpresst. Die so erhaltenen Tablettenkerne wurden mit einem handelsüblichen Filmüberzug auf Wasserbasis enthaltend Hypromellose und TiO₂ befilmt.

Die Filmtabletten weisen eine *in vitro*-Freisetzungsrate des wirkstoffs von 20 % bis 40 % in 3 h, von 44 % bis 64 % in 5 h und von mehr als 80 % in 8,5 h auf.

### Ausführungsbeispiel 8

Es wurden Tabletten mit der in Tabelle 8 angegebenen Zusammensetzung hergestellt:

**Tabelle 8:**

| Bestandteil | Menge pro Tablette | Funktion des Bestandteiles |
|---|---|---|
| Hypromellose (Viskosität 100 mPa·s) | 17.5 mg | Hydrogelbildner |
| Hypromellose (Viskosität 4000 mPa·s) | 25 mg | Hydrogelbildner |
| Mirabegron | 25 mg | Wirkstoff |
| Mikrokristalline Cellulose | 57.5 mg | Füllmittel |
| Siliciumdioxid, hochdisperses Aerosil^{®} 200 | 2 mg | Fließregulierungsmittel |
| Stearinsäure | 1 mg | Schmiermittel |
| | | |
| Endgewicht Tablettenkern | 128 mg | |
| Filmüberzug (Hypromellose, TiO₂) | 3 mg | |
| | | |
| Endgewicht Tablette | 131 mg | |

Die Herstellung der Tabletten erfolgte, indem zunächst die Hypromellosen als Hydrogelbildner, Mirabegron als Wirkstoff, mikrokristalline Cellulose als Füllmittel miteinander vermischt wurden unter Erhalt eines Gemisches. Dieses Gemisch wurde mittels eines Walzenkompaktators komprimiert und anschließend gemahlen und gesiebt. Das so erhaltene Granulat wurde mit Siliciumdioxid als Fließregulierungsmittel und Stearinsäure als Schmiermittel vermischt und das so erhaltene Gemisch zu Tablettenkernen verpresst. Die so erhaltenen Tablettenkerne wurden mit einem handelsüblichen Filmüberzug auf Wasserbasis enthaltend Hypromellose und TiO₂ befilmt.

Die Filmtabletten weisen eine *in vitro*-Freisetzungsrate des Wirkstoffs von 20 % bis 40 % in 3 h, von 44 % bis 64 % in 5 h und von mehr als 80 % in 8,5 h auf.

## Patentansprüche

1. Feste pharmazeutische orale Darreichungsform mit verlängerter Wirkstofffreisetzung, umfassend Mirabegron oder ein pharmazeutisch annehmbares Salz davon als Wirkstoff, wobei der Wirkstoff in einer verlängert freisetzenden Matrix verteilt enthalten ist, die einen Hydrogelbildner umfasst ausgewählt aus der Gruppe bestehend aus auf Poly(meth)acrylat-basierendem Polymer, Alginsäure, Alginat, Carrageenan und einer Mischung von zumindest zwei voneinander verschiedenen Hydroxypropylmethylcellulosen, wobei die Darreichungsform eine *in vitro*-Freisetzungsrate des Wirkstoffs, gemessen unter Anwendung der Drehkörbchen-Apparatur gemäß Eur. Ph. bei 100 U/min in 900 ml Phosphatpuffer mit einem pH-Wert von 6,8 bei einer Temperatur von 37 °C, von 20 % bis 40 % in 3 h, von 44 % bis 64 % in 5 h und von mehr als 80 % in 8,5 h aufweist.

2. Darreichungsform nach Anspruch 1, **dadurch gekennzeichnet, dass** sich die voneinander verschiedenen Hydroxypropylmethylcellulosen hinsichtlich ihrer Viskosität voneinander unterscheiden.

3. Darreichungsform nach einem der voranstehenden Ansprüche, **dadurch gekennzeichnet, dass** das Alginat ein Natriumalginat oder ein Calciumalginat ist.

4. Darreichungsform nach einem der voranstehenden Ansprüche, **dadurch gekennzeichnet, dass** das auf Poly(meth)acrylat-basierende Polymer ein Copolymer von Ethylacrylat und Methylmethacrylat ist oder ein Terpolymer von Ethylacrylat, Methylmethacrylat und einem Methacrylsäureester.

5. Darreichungsform nach einem der voranstehenden Ansprüche, **dadurch gekennzeichnet, dass** die Matrix frei von Polyethylenoxid ist, vorzugsweise die gesamte Darreichungsform.

6. Darreichungsform nach einem der voranstehenden Ansprüche, **dadurch gekennzeichnet, dass** die Matrix frei von Antioxidantien ist, vorzugsweise die gesamte Darreichungsform.

7. Darreichungsform nach einem der voranstehenden Ansprüche, **dadurch gekennzeichnet, dass** das Gewichtsverhältnis von Wirkstoff zu dem Hydrogelbildner in einem Bereich von 1:1 bis 1:50 liegt.

8. Darreichungsform nach einem der voranstehenden Ansprüche, **dadurch gekennzeichnet, dass** die Darreichungsform frei von pharmazeutischen Hilfsstoffen ist, die in Wasser basisch reagieren.

9. Darreichungsform nach einem der voranstehenden Ansprüche, **dadurch gekennzeichnet, dass** die Darreichungsform ferner ein Füllmittel, ein Bindemittel, ein Sprengmittel, ein Schmiermittel und/oder ein Fließregulierungsmittel als pharmazeutisch annehmbaren Hilfsstoff umfasst.

10. Darreichungsform nach einem der voranstehenden Ansprüche, **dadurch gekennzeichnet, dass** die Darreichungsform eine Tablette ist, die optional mit einem Filmüberzug versehen ist.

11. Darreichungsform nach Anspruch 10, **dadurch gekennzeichnet, dass** die Tablette direktkomprimiert ist.

12. Darreichungsform nach Anspruch 10, **dadurch gekennzeichnet, dass** die Tablette ein den Wirkstoff enthaltendes Granulat beinhaltet.

13. Verfahren zur Herstellung einer festen pharmazeutischen oralen Darreichungsform nach einem der Ansprüche 1 bis 12, umfassend die Schritte des
(a) Vermischens des Wirkstoffes mit dem Hydrogelbildner unter Erhalt eines Gemisches;
(b) Verarbeitens des Gemisches aus Schritt (a) zu der Darreichungsform,
wobei das Verfahren unter Ausschluss des Einsatzes eines Lösungsmittels durchgeführt wird.

14. Verfahren nach Anspruch 13, **dadurch gekennzeichnet, dass** das Verfahren die Schritte umfasst
(a) Bereitstellen des Wirkstoffes und des Hydrogelbildners;
(b) Vermischen des Wirkstoffes und des Hydrogelbildners unter Erhalt eines Gemisches; und
(c) Verpressen des Gemisches aus Schritt (b) unter Erhalt eines Tablettenkerns.

15. Verfahren nach Anspruch 13, **dadurch gekennzeichnet, dass** das Verfahren die Schritte umfasst
(a) Bereitstellen des Wirkstoffes und des Hydrogelbildners;
(b) Vermischen des Wirkstoffes und des Hydrogelbildners unter Erhalt eines Gemisches;
(c) Trockengranulieren des Gemisches aus Schritt (b) unter Erhalt eines Trockengranulats; und
(d) Verpressen des Trockengranulats aus Schritt (c) unter Erhalt eines Tablettenkerns.
